(19) Europäisches Patentamt
European Patent Office
Office européen des brevets

(11) Veröffentlichungsnummer: **0 046 735**
**B1**

(12) # EUROPÄISCHE PATENTSCHRIFT

(45) Veröffentlichungstag der Patentschrift: **12.12.84**

(51) Int. Cl.³: **C 07 D 213/61,** C 07 C 121/34, C 07 C 120/00, C 07 C 47/14

(21) Anmeldenummer: **81810344.2**

(22) Anmeldetag: **21.08.81**

(54) **Verfahren zur Herstellung von durch Methyl- oder Trifluormethylgruppen substituierten Chlorpyridinen.**

(30) Priorität: **27.08.80 CH 6447/80**
**11.06.81 CH 3834/81**

(43) Veröffentlichungstag der Anmeldung:
**03.03.82 Patentblatt 82/9**

(45) Bekanntmachung des Hinweises auf die Patenterteilung:
**12.12.84 Patentblatt 84/50**

(84) Benannte Vertragsstaaten:
**AT BE CH DE FR GB IT LI NL**

(56) Entgegenhaltungen:
**EP - A - 0 012 117**
**DE - A - 2 812 607**
**DE - B - 1 136 321**

(73) Patentinhaber: **CIBA-GEIGY AG, Postfach, CH-4002 Basel (CH)**

(72) Erfinder: **Steiner, Eginhard, Dr., Obere Hofackerstrasse 3, CH-4414 Füllinsdorf (CH)**
Erfinder: **Martin, Pierre, Dr., Meisenweg 38, CH-4310 Rheinfelden (CH)**

**EP 0 046 735 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von durch Methyl- oder Trifluormethylgruppen substituierten Chlorpyridinen sowie die dabei intermediär hergestellten oder entstehenden neuen Zwischenprodukte.

Durch Methyl- oder Trifluormethylgruppen substituierten Chlorpyridine konnten bisher nur durch aufwendige mehrstufige Verfahren hergestellt werden.

2,5-Dichlor-3-methylpyridin und 2,3-Dichlor-5-methylpyridin können z. B. dadurch erhalten werden, daß man 2-Chlor-3-methyl-5-aminopyridin bzw. 2-Chlor-3-amino-5-methylpyridin diazotiert und unter Diazospaltung mit Chlor substituiert. Die genannten Aminopyridine können durch Chlorierung von 3-Methylpyridin zum 2-Chlor-3-methylpyridin und 2-Chlor-5-methylpyridin, Nitrierung zum 2-Chlor-3-methyl-5-nitropyridin bzw. 2-Chlor-3-nitro-methylpyridin und Reduktion der Nitroverbindungen hergestellt werden. Bei der Chlorierung des 3-Methylpyridins entstehen im allgemeinen neben der gewünschten Verbindung mehrere Isomere. Durch Chlorierung des 2,3-Dichlor-5-methylpyridins erhält man das 2,3-Dichlor-5-trichlormethylpyridin das durch Austausch der Chloratome der Trichlormethylgruppe gegen Fluoratome in das 2,3-Dichlor-5-trifluormethyl-pyridin überführt werden kann (vgl. z. B. die europäische Patentveröffentlichung 004 414). Durch Erhitzen von 2,4-Dimethylpyrrol und Chloroform in der Gasphase bei Temperaturen von ca. 550°C erhält man neben 5 anderen Isomeren das 2-Chlor-3,5-dimethylpyridin [vgl. J. Chem. Soc. Perkin Trans. I, 1578–82 (1979)].

Es wurde gefunden, daß man Chlorpyridine der Formel I

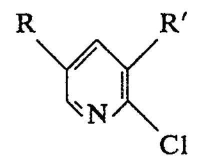

(I)

worin entweder R Chlor und R′ Methyl oder Trifluormethyl oder R und R′ Methyl bedeuten, auf sehr einfache, wirtschaftliche und umweltfreundliche Weise in guten Ausbeuten und unter Verwendung von leicht zugänglichen, billigen Ausgangsmaterialien in der Weise herstellen kann, daß man in Gegenwart eines Katalysators

a) Trichloracetaldehyd an Methylacrylonitril oder α-Trifluormethylacrylonitril oder
b) 2,2-Dichlorpropionaldehyd an Methacrylnitril

anlagert und das gebildete Zwischenprodukt der Formel II

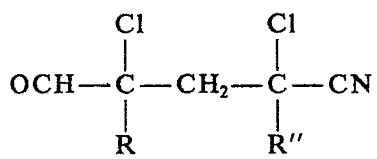

(II)

worin entweder R Chlor und R″ Methyl oder Trifluormethyl oder R und R″ Methyl bedeuten, zu einer Verbindung der Formel I cyclisiert.

Eine Reaktionsfolge von nicht alkylsubstituiertem Trichlorformylbutyronitril zu 2,3,5-Trichlorpyridin unter Wasserabspaltung ist in der europäischen Patentveröffentlichung Nr. 12 117 beschrieben. Die Cyclisierung unter Aromatisierung zu chlorierten Methyl- bzw. Trifluormethylpyridinen der Formel I aus chlorierten und methyl- bzw. trifluormethylsubstituierten Formylbutyronitrilen der Formel II unter HOCl-Abspaltung war jedoch nicht vorauszusehen. Die erfindungsgemäße Reaktionsfolge ist daher als in hohem Maße überraschend zu bezeichnen. Die Bildung der Additionsverbindungen der Formel II aus 2,2-Dichlorpropionaldehyd in gleicher Weise überraschend, da die reaktiven Eigenschaften von Trichloracetaldehyd von denjenigen seiner Homologen sehr verschieden sind [vgl. z. B. Chem. Ber. 97, 3322 (1964)], insbesondere wird Chlorbeweglichkeit am Chloracetaldehyd durch einen zusätzlichen Kohlenstoffsubstituenten wie Methyl, Trichlormethyl oder Trifluormethyl erheblich eingeschränkt.

Die Anlagerungsreaktionen können in offenem oder geschlossenem System, bevorzugt bei einer Temperatur von 70–160°C, durchgeführt werden. Bevorzugt erfolgt die Anlagerung in geschlossenem System bei einem der angewendeten Reaktionstemperatur entsprechenden Druck, der beispielsweise im Bereich von 1–30 bar liegen kann.

Als Katalysatoren für die Anlagerungsreaktionen können erfindungsgemäß Metalle der Hauptgruppe VIII und der Nebengruppe VIa, VIIa, Ib und IIb des periodischen Systems, z. B. Eisen, Kobalt, Nickel, Ruthenium, Palladium, Chrom, Molybdän, Mangan, Kupfer und Zink verwendet werden. Diese Metalle können in elementarer Form oder in Form von Verbindungen eingesetzt werden. Geeignete Verbindungen sind beispielsweise Oxide und Salze, wie Halogenide, Sulfate, Sulfite, Sulfide, Nitrate, Acetate, Stearate, Citrate, Carbonate, Cyanide und Rhodanide, sowie Komplexe mit Liganden, wie Phosphinen, Phosphiten, Benzoyl- und Acetylacetonaten, Nitrilen, Isonitrilen und Kohlenmonoxid.

Als Beispiele seien genannt: Kupfer(II)oxid, Eisen(III)oxid; Kupfer(I)-, Kupfer(II)-, Eisen(II)- und

Eisen(III)bromide, -jodide und vor allem -chloride, Zinkchlorid, sowie die Chloride des Rutheniums, Rhodiums, Palladiums, Kobalts und Nickels; Kupfer(II)sulfat, Eisen(II)- und Eisen(III)sulfat; Kupfer(II)-nitrat und Eisen(III)nitrat; Mangan(III)acetat, Kupfer(II)acetat, Kupfer(II)stearat, Eisen(III)citrat, Kupfer(I)cyanid; Ruthenium(II)dichlor-tris-triphenylphosphin, Rhodium-dichloro-tris-triphenylphosphin; Chrom- und Nickelacetylacetonat, Kupfer(II)acetylacetonat, Eisen(III)acetylacetonat, Kobalt(II)- und Kobalt(III)acetylacetonat, Mangan(II)acetylacetonat, Kupfer(II)benzoylacetonat; Eisencarbonyl-cyclopentadienylkomplex; Molybdäncarbonylcyclopentadienylkomplex, Chromtricarbonylarylkomplexe, Ruthenium(II)acetatkomplex, Chrom- und Molybdänhexacarbonyl, Nickeltetracarbonyl, Eisenpentacarbonyl, Kobalt- und Mangancarbonyl.

Es können auch Gemische der genannten Metalle mit Metallverbindungen und/oder anderen Zusätzen verwendet werden, wie Kupferpulver in Kombination mit einer der vorerwähnten Kupferverbindungen; Gemische von Kupferpulver mit Lithiumhalogeniden, wie Lithiumchlorid oder Isocyaniden, wie tert.-Butylisocyanid; Gemische von Eisenpulver mit Eisen(III)chlorid, gegebenenfalls unter Zusatz von Kohlenmonoxid; Gemische von Eisen(III)chlorid mit Benzoin; Gemische von Eisen(II)- oder Eisen(III)-chlorid mit Trialkylphosphiten; Gemische von Eisenpentacarbonyl und Jod.

Bevorzugt sind Eisen(II)- und Eisen(III)salze und -komplexe, vor allem Eisen(II)- und Eisen(III)chlorid, sowie Eisenpulver; Ruthenium(III)chlorid, Ruthenium(II)dichloro-tris-triphenylphosphin, Kupferpulver, Kupferbronze, Kupfer(I)- und Kupfer(II)salze und -komplexe, wie Kupfer(I)chlorid, Kupfer(II)chlorid, Kupfer(I)bromid, Kupfer(II)bromid; Kupfer(II)acetat, Kupfer(II)acetylacetonat, Kupfer(II)benzoylacetonat, Kupfer(II)sulfat, Kupfer(II)nitrat, Kupfer(I)cyanid und Kupfer(I)jodid.

Ganz besonders bevorzugt sind Kupferpulver, Kupferbronze, Kupfer(I)- und Kupfer(II)chlorid bzw. -bromid und Kupfer(I)jodid, sowie deren Gemische.

Die Katalysatoren werden im allgemeinen in Mengen von etwa 0,01 bis 10 Mol%, bevorzugt 0,1 bis 5 Mol%, bezogen auf den Aldehyd, verwendet.

Die Anlagerung der definitionsgemäßen Aldehyde an das Acrylonitril bzw. Methacrylonitril oder $\alpha$-Trifluormethylacrylonitril wird zweckmäßig in Gegenwart eines inerten organischen Lösungsmittels vorgenommen. Geeignete Lösungsmittel sind solche, in denen die Katalysatoren ausreichend löslich sind oder die mit den Katalysatoren Komplexe bilden können, die aber gegenüber den Reaktionspartnern inert sind. Als Beispiele für geeignete Lösungsmittel seien genannt: Alkancarbonsäurenitrile, insbesondere solche mit 2—5 Kohlenstoffatomen, wie Acetonitril, Propionitril und Butyronitril; 3-Alkoxypropionitrile mit 1—2 Kohlenstoffatomen in der Alkoxygruppe, wie 3-Methoxypropionitril und 3-Äthoxypropionitril; aromatische Nitrile, vor allem Benzonitril; aliphatische Ketone mit vorzugsweise insgesamt 3—8 Kohlenstoffatomen, wie Aceton, Diäthylketon, Methylisopropylketon, Diisopropylketon, Methyl-tert.-butylketon; Alkyl- und Alkoxyalkylester von aliphatischen Monocarbonsäuren mit insgesamt 2—6 Kohlenstoffatomen, wie Ameisensäuremethyl- und -äthylester, Essigsäuremethyl-, -äthyl-, -n-butyl- und -isobutylester, sowie l-Acetoxy-2-methoxyäthan; cyclische Äther, wie Tetrahydrofuran, Tetrahydropyran und Dioxan; Dialkyläther mit je 1—4 Kohlenstoffatomen in den Alkylgruppen, wie Diäthyläther, Di-n-propyläther und Diisopropyläther; N,N-Dialkylamide von Alkancarbonsäuren mit 1—3 Kohlenstoffatomen in der Alkylgruppe, wie N,N-Dimethylformamid, N,N-Dimethylacetamid, N,N-Diäthylacetamid und N,N-Dimethylmethoxyacetamid; Äthylenglykol- und Diäthylenglykoldialkyläther mit je 1—4 Kohlenstoffatomen in den Alkylgruppen, wie Äthylenglykoldimethyl-, -diäthyl- und -di-n-butyläther; Diäthylenglykoldiäthyl- und -di-n-butyläther; Phosphorsäure-tris-N,N-dimethylamid (Hexametapol). Ferner können überschüssiges Acrylonitril bzw. Methacrylonitril oder $\alpha$-Trifluormethylacrylonitril als Lösungsmittel verwendet werden.

Bevorzugte Lösungsmittel für die Anlagerungsreaktionen sind Alkancarbonsäurenitrile mit 2—5 Kohlenstoffatomen und 3-Alkoxypropionitrile mit 1—2 Kohlenstoffatomen in der Alkoxygruppe, insbesondere Acetonitril, Butyronitril und 3-Methoxypropionitril, oder die als Reaktanden verwendeten ungesättigten Nitrile.

Die Zwischenprodukte der Formel II sind neu und speziell für die Synthese der Verbindungen der Formel I entwickelt worden und bilden deshalb ebenfalls einen Gegenstand der Erfindung. Bevorzugt sind Verbindungen der Formel II, worin R Chlor und R″ Methyl darstellen.

Die Cyclisierung der Verbindungen der Formel II kann in einem offenen oder einem geschlossenen System bei Temperaturen zwischen etwa 0 und 220°C, insbesondere zwischen etwa 100 und 200°C, durchgeführt werden. Vorzugsweise wird die Cyclisierung in einem offenen System durchgeführt. Bei der Cyclisierung in einem offenen System ist es vorteilhaft, diese in Gegenwart von Chlorwasserstoff oder in Gegenwart von Substanzen, welche unter den Reaktionsbedingungen Chlorwasserstoff bilden, wie Phosgen, Bortrichlorid, Aluminiumchlorid, Trialkylammoniumchloriden mit je 1—4 Kohlenstoffatomen in den Alkylgruppen, Phosphorpentachlorid, Phosphoroxychlorid oder Phosphortrichlorid, vorzunehmen. Vorzugsweise wird die Cyclisierung in Gegenwart von Chlorwasserstoff vorgenommen.

Die Cyclisierung wird bevorzugt ohne Zusatz eines Lösungsmittels, in der Flüssig- oder in der Gasphase durch bloßes Erhitzen der Verbindungen der Formel II durchgeführt. Sie kann aber auch in Gegenwart eines organischen Lösungsmittels vorgenommen werden. Als organische Lösungsmittel eignen sich beispielsweise chlorierte aliphatische Kohlenwasserstoffe, wie Chloroform, Methylenchlorid und Tetrachloräthan; gegebenenfalls chlorierte aromatische Kohlenwasserstoffe, wie Benzol,

Toluol, Xylole und Chlorbenzole; N,N-Dialkylamide von Alkancarbonsäuren mit 1—3 Kohlenstoffatomen, wie N,N-Dimethylformamid, N,N-Dimethylacetamid, N,N-Diäthylacetamid und N,N-Dimethylmethoxyacetamid; cyclische Amide, wie N-Methyl-2-pyrrolidon, N-Acetyl-2-pyrrolidon und N-Methyl-$\varepsilon$-caprolactam; Amide der Kohlensäure, wie Tetramethylharnstoff und Dimorpholinocarbonyl; Amide der phosphorigen Säure, der Phosphorsäure, der Phenylphosphonsäure oder von Alkylphosphonsäuren mit 1—3 Kohlenstoffatomen in der Alkylgruppe, wie Phosphorsäuretriamid, Phosphorsäure-tris-(N,N-dimethylamid), Phosphorsäuretrimorpholid, Phosphorsäuretripyrrolinid, Phosphorigsäure-tris-(N,N-dimethylamid), Methanphosphonsäure-bis-(N,N-dimethylamid); Amide der Schwefelsäure oder von aliphatischen oder aromatischen Sulfonsäuren, wie Tetramethylsulfamid, Methansulfonsäuredimethylamid, oder p-Toluolsulfonsäureamid; aliphatische Ketone, cyclische Äther, Dialkyläther sowie Äthylenglykol- und Diäthylenglykoldialkyläther der vorerwähnten Art, sowie Phosphortrichlorid und Phosphoroxychlorid.

Bevorzugte Lösungsmittel für die Cyclisierung sind Chloroform, Methylenchlorid, cyclische Äther und Dialkyläther mit je 1—4 Kohlenstoffatomen in den Alkylgruppen, insbesondere Dioxan und Diäthyläther, sowie N,N-Dialkylamide von Alkancarbonsäuren mit 1—3 Kohlenstoffatomen, insbesondere N,N-Dimethylformamid.

Das erfindungsgemäße Verfahren kann vorteilhaft in der Weise durchgeführt werden, daß man die durch die Anlagerung gebildeten Verbindungen der Formel II zunächst isoliert und anschließend in einer zweiten Verfahrensstufe cyclisiert. Dabei werden die einzelnen Verfahrensstufen wie vorstehend beschrieben durchgeführt.

Gemäß einer vorteilhaften Ausführungsform des erfindungsgemäßen Verfahrens werden die definitionsgemäßen Aldehyde bei einer Temperatur von 70—160°C in Acetonitril, Butyronitril oder 3-Methoxypropionitril als Lösungsmittel in Gegenwart von 0,1—5 Mol% Kupferpulver, Kupferbronze-, Kupfer(I)- oder Kupfer(II)chlorid bzw. -bromid oder Kupfer(I)jodid oder in Gegenwart eines Gemisches dieser Substanzen in einem geschlossenen System mit Methacrylonitril bzw. $\alpha$-Trifluormethylacrylonitril umgesetzt, und die nach Abtrennung des Lösungsmittels erhaltenen Verbindungen der Formel II werden bei einer Temperatur zwischen 100 und 200°C in einem offenen System in Gegenwart von Chlorwasserstoff oder einer Substanz, welche unter den Reaktionsbedingungen Chlorwasserstoff bildet, zu Verbindungen der Formel I cyclisiert.

Man kann jedoch auch auf die Isolierung der Zwischenprodukte der Formel II verzichten und die Additions- und die Cyclisierungsreaktion in einem Arbeitsgang durchführen. In diesem Fall erfolgt die Umsetzung der definitionsgemäßen Aldehyde mit Methacrylonitril oder $\alpha$-Trifluormethylacrylonitril zu den Chlorpyridinen der Formel I bevorzugt bei einer Temperatur zwischen 70 und 220°C, insbesondere zwischen 130 und 200°C. Dabei kann in offenem oder geschlossenem System gearbeitet werden. Wird die Umsetzung in offenem System durchgeführt, so kann es zweckmäßig sein, diese in Gegenwart von Chlorwasserstoff oder in Gegenwart von Substanzen vorzunehmen, welche unter den Reaktionsbedingungen Chlorwasserstoff bilden. Derartige Substanzen sind beispielsweise Phosgen, Bortrichlorid, Aluminiumchlorid, Trialkylammoniumchloride mit je 1—4 Kohlenstoffatomen in den Alkylgruppen, Phosphorpentachlorid, Phosphoroxychlorid oder Phosphortrichlorid. Die einstufige Herstellung von Chlorpyridinen der Formel I erfolgt vorzugsweise in einem geschlossenen System bei einem der jeweiligen Reaktionstemperatur entsprechenden Druck, der beispielsweise je nach Reaktionstemperatur im Bereich von 1—50 bar liegen kann. Besonders bevorzugt ist die einstufige Synthese von Verbindungen der Formel I in einem geschlossenen System bei einem Druck von 1—30 bar.

Diese einstufige Synthese wird ebenfalls in Gegenwart eines Katalysators und zweckmäßig in Gegenwart eines inerten organischen Lösungsmittels durchgeführt. Als Katalysatoren und Lösungsmittel kommen solche der eingangs beschriebenen Art in Betracht, wobei in bezug auf bevorzugte Katalysatoren und Katalysatormengen das im Vorangehenden Erwähnte gilt.

Bevorzugte Lösungsmittel für die einstufige Durchführung des Verfahrens sind Alkancarbonsäurenitrile mit 2—5 Kohlenstoffatomen und 3-Alkoxypropionitrile mit 1—2 Kohlenstoffatomen in der Alkoxygruppe. Besonders geeignete Lösungsmittel sind Acetonitril, Butyronitril und 3-Methoxypropionitril oder ein Überschuß der als Reaktanden verwendeten ungesättigten Nitrile. Nach Beendigung der Reaktion können die Chlorpyridine der Formel I auf übliche Weise, z. B. durch Abdampfen des Lösungsmittels und Reinigung des Rohprodukts durch Destillation oder eventuell Wasserdampfdestillation, isoliert werden.

Gemäß einer weiteren vorteilhaften Ausführungsform des erfindungsgemäßen Verfahrens setzt man die Aldehyde und das Methacrylonitril bzw. $\alpha$-Trifluormethylacrylonitril in Acetonnitril, Butyronitril oder 3-Methoxypropionitril als Lösungsmittel in Gegenwart von 0,1 bis 5 Mol% Kupferpulver, Kupferbronze Kupfer(I)- oder Kupfer(II)chlorid bzw. -bromid oder Kupfer(I)jodid oder einem Gemisch dieser Substanzen bei 130—200°C in einem geschlossenen System bei einem der jeweils angewandten Reaktionstemperatur entsprechenden Druck direkt zu den Chlorpyridinen der Formel I um.

Die Chlorpyridine der Formel I können auf an sich bekannte Weise über eine oder mehrere Zwischenstufen zur Herstellung verschiedener Wirkstoffe, besonders von Insektiziden und Herbiziden, verwendet werden (vgl. schweizerisches Patent 622 170, europäische Patentveröffentlichungen Nr. 00176, 00483 und 04414; europäisches Patent 40179; DE-OS 28 12 649 und 2 748 636; Südafr. Patent 7 802 440; japanische Patentpublikationen 5 4115—380, 5 5038—356, 5 5079—369 und

56–39069 und belgische Patentschrift 862 325).

Das erfindungsgemäße Verfahren wird durch die folgenden Beispiele näher erläutert.

### Beispiel 1

#### a) Herstellung von 4-Formyl-2-methyl-2,4,4-trichlorbutyronitril

14,7 g Trichloracetaldehyd, 13,5 g Methacrylonitril und 0,3 g Kupferpulver (aktiviert nach dem in Org. Synth. Coll, Vol. III, 339, für Kupferbronze angegebenen Verfahren) werden mit 30 ml Acetonitril in einem Emaille-Autoklaven während 15 Stunden auf 100°C erhitzt. Nach dem Erkalten wird das Lösungsmittel am Wasserstrahlvakuum bei ca. 40–50°C abdestilliert. Der Rückstand wird mit 50 ml Diäthyläther versetzt, und der ausgefallene Kupferschlamm wird abfiltriert. Nach dem Abdestillieren des Diäthyläthers wird der Rückstand im Hochvakuum rektifiziert. Die bei 76–78°C und 13 Pa siedende Fraktion wird aufgefangen. Man erhält 13,8 g 4-Formyl-2-methyl-2,4,4-trichlorbutyronitril in Form eines farblosen Öls.

IR-Spektrum (flüssig) in $cm^{-1}$: 2250 (CN), 1750 (CO).

$^1H$-NMR-Spektrum (60 MHz in $CDCl_3$) in ppm: 9,30 (s, 1H, –CHO); 3,22 (s, 2H, $H_2$ an C–3); 2,60 (s, 3H, $-CH_3$).

Elementaranalyse für $C_6H_6Cl_3NO$ (Molgewicht 214,48):

| | | | | |
|---|---|---|---|---|
| berechnet | C 33,60% | H 2,82% | N 6,53% | Cl 49,59% |
| gefunden | C 34,1% | H 3,1% | N 6,8% | Cl 48,6% |

#### b) Herstellung von 2,5-Dichlor-3-methylpyridin

21,4 g des gemäß Beispiel 1 a) erhaltenen 4-Formyl-2-methyl-2,4,4-trichlorbutyronitrils werden unter Durchleitung eines schwachen Stromes trockenen HCl-Gases während 4–5 Stunden auf 145°C erhitzt. Nach dem Erkalten wird die dunkle Schmelze einer Wasserdampfdestillation unterworfen. Man erhält 9,9 g 2,5-Dichlor-3-methylpyridin in Form von farblosen Kristallen. Fp. 42°C (umkristallisiert aus $CH_3OH/H_2O$ im Volumenverhältnis 4 : 1).

$^1H$-NMR-Spektrum (60 MHz in $CDCl_3$) in ppm: 8,15 (d, 1H, H an C–6); 7,50 (d, 1H, H an C–4); 2,40 (s, 3H, $-CH_3$).

Elementaranalyse für $C_6H_5Cl_2N$ (Molgewicht 162,02):

| | | | | |
|---|---|---|---|---|
| berechnet | C 44,48% | H 3,11% | N 8,65% | Cl 43,77% |
| gefunden | C 44,4% | H 2,9% | N 7,9% | Cl 53,8% |

### Beispiel 2

#### Einstufige Herstellung von 2,5-Dichlor-3-methylpyridin

14,7 g Trichloracetaldehyd, 13,5 g Methacrylonitril, 0,5 g Kupfer(I)chlorid und 40 ml Acetonitril werden in einem Tantal-Autoklaven während 2 Stunden auf 150°C und hierauf während weiterer 2 Stunden auf 180°C erhitzt. Nach dem Abdestillieren des Lösungsmittels wird der Rückstand mit 50 ml Diäthyläther versetzt und filtriert. Nach dem Abdestillieren des Diäthyläthers am Wasserstrahlvakuum wird der Rückstand einer Wasserdampfdestillation unterworfen. Das erhaltene kristalline Produkt ist mit der gemäß Beispiel 1 b) erhaltenen Verbindung identisch.

### Beispiel 3

#### Einstufige Herstellung von 2-Chlor-3,5-dimethylpyridin

12,7 g 2,2-Dichlorpropionaldehyd, 10,0 g Methacrylonitril, 0,5 g Kupfer(I)chlorid und 40 ml Acetonitril werden in einem Tantal-Autoklaven während 2 Stunden auf 180°C erhitzt. Der Autoklaveninhalt wird nach dem Erkalten in 50 ml Diäthyläther aufgenommen, filtriert und das Filtrat wird im Vakuum eingedampft. Der Rückstand wird einer Wasserdampfdestillation unterworfen. Das erhaltene kristalline Produkt ist mit der gemäß Beispiel 4 b) erhaltenen Substanz identisch.

Beispiel 4

a) Herstellung von 4-Formyl-2-methyl-2,4-dichlorvaleriansäurenitril

12,7 g 2,2-Dichlorpropionaldehyd, 13,5 g Methacrylonitril, 0,5 g Kupfer(I)chlorid und 40 ml Acetonitril werden in einem Tantal-Autoklaven während einer Stunde auf 130°C und hierauf während 2 Stunden auf 150°C erhitzt. Nach dem Abdestillieren des Lösungsmittels und des überschüssigen Methacrylonitrils am Wasserstrahlvakuum wird der Rückstand in 50 ml Diäthyläther aufgenommen und filtriert. Der Diäthyläther wird im Vakuum abdestilliert und der Rückstand wird im Hochvakuum rektifiziert. Die bei 76—77°C und 13 Pa siedende Fraktion wird aufgefangen. Man erhält 10,8 g 4-Formyl-2-methyl-2,4-dichlorvaleriansäurenitril in Form eines hellbraunen Öls.

IR-Spektrum (flüssig) in $cm^{-1}$: 2250 (CN), 1750 (CO).

$^1$H-NMR-Spektrum (60 MHz in $CDCl_3$) in ppm: (Diastereomerengemisch im Verhältnis 1 : 1) 9,71 und 9,53 je (s, 1H, —CHO); 2,96 (s, 4H, 2 x—$CH_2$); 2,14 (s, 6H, 2 x—$CH_3$); 2,02 (s, 3H, —$CH_3$); 1,93 (s, 3H, —$CH_3$).

Elementaranalyse für $C_7H_9Cl_2NO$ (Molgewicht 194,06):

| | | | | |
|---|---|---|---|---|
| berechnet | C 43,22% | H 4,67% | N 7,21% | Cl 36,53% |
| gefunden | C 43,6% | H 4,6% | N 7,3% | Cl 35,9% |

b) Herstellung von 2-Chlor-3,5-dimethylpyridin

19,4 g des gemäß Beispiel 4 a) erhaltenen 4-Formyl-2-methyl-2,4-dichlorvaleriansäurenitrils werden unter Durchleitung eines schwachen Stroms trockenen HCl-Gases während 4 Stunden auf 160—170°C erhitzt. Nach dem Erkalten wird die dunkle Schmelze einer Wasserdampfdestillation unterworfen. Das Destillat wird mit Diäthyläther ausgeschüttelt, dieser wird getrocknet und im Vakuum eingedampft. Das zurückbleibende hellbraune Öl wird destilliert. Man erhält 7,36 g 2-Chlor-3,5-dimethylpyridin in Form eines bei 110°C und 2500 Pa siedenden hellbraunen Öls.

$^1$H-NMR-Spektrum (60 MHz in $CDCl_3$) in ppm: 8,0 (d, 1 H, H an C—6); 7,31 (d, 1 H, H an C—4, $J_{6-4} = 2,0$ Hz); 2,34 (s, 3H, —$CH_3$); 2,28 (s, 3H, —$CH_3$).

Elementaranalyse für $C_7H_8ClN$ (Molgewicht 141,60):

| | | | | |
|---|---|---|---|---|
| berechnet | C 59,38% | H 5,65% | N 9,83% | Cl 25,04% |
| gefunden | C 59,1% | H 5,9% | N 9,7% | Cl 25,3% |

Die Verbindung ist nach den $^1$H-NMR-Daten identisch mit dem in J. Chem. Soc. Perkin I (1979), 1578, beschriebenen 2-Chlor-3,5-lutidin.

**Patentansprüche**

1. Verfahren zur Herstellung von Chlorpyridinen der Formel I

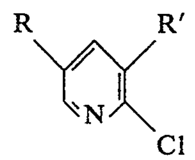

(I)

worin entweder R Chlor und R′ Methyl oder Trifluormethyl oder R und R′ Methyl bedeuten, dadurch gekennzeichnet, daß man in Gegenwart eines Katalysators

a) Trichloracetaldehyd an Methylacrylonitril oder $\alpha$-Trifluormethylacrylonitril oder

b) 2,2-Dichlorpropionaldehyd an Methacrylonitril

anlagert und das gebildete Zwischenprodukt der Formel II

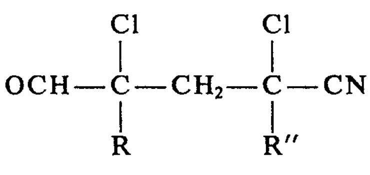

(II)

worin entweder R Chlor und R″ Methyl oder Trifluormethyl oder R und R″ Methyl bedeuten, zu einer Verbindung der Formel I cyclisiert.

2. Verfahren nach Anspruch 1, dadurch gekennzeichnet, daß man die Anlagerungsreaktionen bei einer Temperatur von 70–160°C durchführt.

3. Verfahren nach Anspruch 1, dadurch gekennzeichnet, daß man die Anlagerungsreaktionen in einem geschlossenen System bei einer Temperatur von 70–160°C und einem der angewendeten Reaktionstemperatur entsprechenden Druck durchführt.

4. Verfahren nach Anspruch 1, dadurch gekennzeichnet, daß man die Cyclisierung der Verbindungen der Formel II bei einer Temperatur zwischen 0 und 220°C durchführt.

5. Verfahren nach Anspruch 1, dadurch gekennzeichnet, daß man die Cyclisierung der Verbindungen der Formel II bei einer Temperatur zwischen 100 und 200°C in einem offenen System in Gegenwart von Chlorwasserstoff oder in Gegenwart von Substanzen, welche unter den Reaktionsbedingungen Chlorwasserstoff bilden, durchführt.

6. Verfahren nach Anspruch 1, dadurch gekennzeichnet, daß man die Cyclisierung der Verbindungen der Formel II in Abwesenheit eines Lösungsmittels durch Erhitzen in der Flüssig- oder in der Gasphase vornimmt.

7. Verfahren nach Anspruch 1, dadurch gekennzeichnet, daß man die Verbindungen der Formel II zunächst isoliert und anschließend in einer zweiten Verfahrensstufe cyclisiert.

8. Verfahren nach Anspruch 1, dadurch gekennzeichnet, daß man in Gegenwart eines Katalysators
a) Trichloracetaldehyd und Methacrylonitril oder α-Trifluormethacrylonitril oder
b) 2,2-Dichlorpropionaldehyd und Methacrylonitril ohne Isolierung der intermediär gebildeten Verbindung der Formel II
direkt zu einer Verbindung der Formel I umsetzt.

9. Verfahren nach Anspruch 8, dadurch gekennzeichnet, daß man die Umsetzung bei einer Temperatur zwischen 70 und 220°C durchführt.

10. Verfahren nach Anspruch 8, dadurch gekennzeichnet, daß man die Umsetzung in einem geschlossenen System bei einem der jeweiligen Reaktionstemperatur entsprechenden Druck durchführt.

11. Verfahren nach Anspruch 1 oder 8, dadurch gekennzeichnet, daß man als Katalysator Kupferpulver, Kupferbronze, Kupfer(I)- oder Kupfer(II)chlorid bzw. -bromid, Kupfer(I)jodid oder deren Gemische verwendet.

12. Verfahren nach Anspruch 1 oder 8, dadurch gekennzeichnet, daß man den Katalysator in Mengen von 0,01 bis 10 Mol%, bezogen auf den Aldehyd, verwendet.

13. Verfahren nach Anspruch 1 oder 8, dadurch gekennzeichnet, daß man die Anlagerungsreaktion bzw. die direkte Umsetzung zu Verbindungen der Formel I in Gegenwart eines inerten organischen Lösungsmittels durchführt.

14. Verfahren nach Anspruch 1 oder 8, dadurch gekennzeichnet, daß man die Anlagerungsreaktionen bzw. die direkte Umsetzung zu Verbindungen der Formel I in einem Alkancarbonsäurenitril mit 2–5 Kohlenstoffatomen, einem 3-Alkoxypropionitril mit 1–2 Kohlenstoffatomen in der Alkoxygruppe oder in überschüssigem Methacrylonitril oder α-Trifluormethacrylonitril als Lösungsmittel durchführt.

15. Verfahren nach Anspruch 1 oder 13, dadurch gekennzeichnet, daß man die Anlagerungsreaktionen bei einer Temperatur von 70–160°C in Acetonitril, Butyronitril oder 3-Methoxypropionitril als Lösungsmittel in Gegenwart von 0,1–5 Mol% Kupferpulver, Kupferbronze, Kupfer(I)- oder Kupfer(II)chlorid bzw. -bromid oder Kupfer(I)jodid oder in Gegenwart eines Gemisches dieser Substanzen in einem geschlossenen System durchführt und die erhaltene Verbindung der Formel II bei einer Temperatur zwischen 100 und 200°C in einem offenen System in Gegenwart von Chlorwasserstoff oder einer Substanz, welche unter den Reaktionsbedingungen Chlorwasserstoff bildet, zu einer Verbindung der Formel I cyclisiert.

16. Verfahren nach Anspruch 8 oder 13, dadurch gekennzeichnet, daß man die Umsetzung in Acetonitril, Butyronitril oder 3-Methoxypropionitril als Lösungsmittel in Gegenwart von 0,1–5 Mol% Kupferpulver, Kupferbronze, Kupfer(I)- oder Kupfer(II)chlorid bzw. -bromid oder Kupfer(I)jodid oder einem Gemisch dieser Substanzen bei 130 bis 200°C in einem geschlossenen System bei einem der jeweils angewendeten Reaktionstemperatur entsprechenden Druck vornimmt.

17. Verbindungen der Formel II

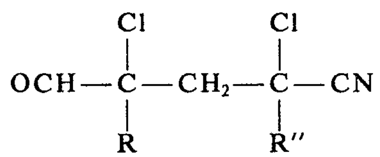

(II)

worin entweder R Chlor und R'' Methyl oder Trifluormethyl, oder R und R'' Methyl bedeuten.

18. Verfahren zur Herstellung einer Verbindung der Formel II nach Anspruch 17, dadurch gekennzeichnet, daß man in Gegenwart eines Katalysators
a) Trichloracetaldehyd an Methacrylonitril oder α-Trifluormethylacrylonitril oder
b) 2,2-Dichlorpropionaldehyd an Methacrylonitril
anlagert.

**Claims**

1. A process for the production of a chloropyridine of the formula I

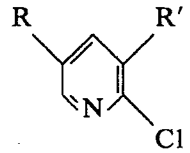

, (I)

wherein either R is chlorine and R′ is methyl or trifluormethyl, or R and R′ are methyl, which process comprises the addition of

a) trichloroacetaldehyde to methacrylonitrile or $\alpha$-trifluoromethacrylonitrile,
b) 2,2-dichloropropionaldehyde to methacrylonitrile,

in the presence of a catalyst, and cyclising the intermediate of the formula II

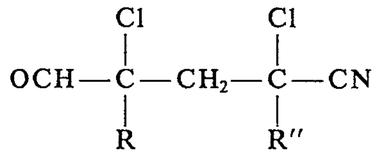

(II)

wherein either R is chlorine and R″ is methyl or trifluoromethyl, or R and R″ are methyl, to give a compound of the formula I.

2. A process according to claim 1, wherein the addition reaction is carried out in the temperature range from 70° to 160°C.

3. A process according to claim 1, wherein the addition reaction is carried out in a closed system in the temperature range from 70° to 160°C and under a pressure corresponding to the reaction temperature.

4. A process according to claim 1, wherein the cyclisation of the compound of the formula II is carried out in the temperature range from 0° to 220°C.

5. A process according to claim 1, wherein the cyclisation of the compound of the formula II is carried out in the temperature range from 100° to 200°C in an open system and in the presence of hydrogen chloride or of a substance which forms hydrogen chloride under the reaction conditions.

6. A process according to claim 1, wherein the cyclisation of the compound of the formula II is carried out in the absence of a solvent, by heating in the liquid or in the gas phase.

7. A process according to claim 1, wherein the compound of the formula II is first isolated and then cyclised in a second step.

8. A process according to claim 1, wherein

a) trichloroacetaldehyde and methacrylonitrile or $\alpha$-trifluoromethacrylonitrile, or
b) 2,2-dichloropropionaldehyde and methacrylonitrile,

are reacted, in the presence of a catalyst, direct without isolation of the intermediate of the formula II, to give a compound of the formula I.

9. A process according to claim 8, wherein the reaction is carried out in the temperature range from 70° to 220°C.

10. A process according to claim 8, wherein the reaction is carried out in a closed system under a pressure corresponding to the respective reaction temperature.

11. A process according to either of claims 1 or 8, wherein the catalyst is copper powder, copper bronze, copper(I) or copper(II) chloride or bromide, copper(I) iodide, or a mixture thereof.

12. A process according to either of claims 1 or 8, wherein the catalyst is employed in an amount of 0.01 to 10 mol.%, based on the aldehyde.

13. A process according to either of claims 1 or 8, wherein the addition reaction or the direct reaction to give a compound of the formula I is carried out in the presence of an inert organic solvent.

14. A process according to either of claims 1 or 8, wherein the addition reaction or the direct reaction to give a compound of the formula I is carried out in an alkanecarboxylic acid nitrile containing 2 to 5 carbon atoms, a 3-alkoxypropionitrile containing 1 to 2 carbon atoms in the alkoxy moiety, or in an excess of acrylonitrile, methacrylonitrile or $\alpha$-trifluoromethacrylonitrile, as solvent.

15. A process according to either of claims 1 or 13, wherein the addition reaction is carried out in the temperature range from 70° to 160°C in acetonitrile, butyronitrile or 3-methoxypropionitrile as solvent, in the presence of 0.1 to 5 mol.% of copper powder, copper bronze, copper(I) or copper(II) chloride or bromide or copper(I) iodide, or in the presence of a mixture of said substances, in a closed system, and

the compound of the formula II obtained as intermediate is cyclised to a compound of the formula I in the temperature range from 100° to 200°C, in an open system, and in the presence of hydrogen chloride or of a substance which forms hydrogen chloride under the reaction conditions.

16. A process according to either of claims 8 or 13, wherein the reaction is carried out in acetonitrile, butyronitrile or 3-methoxypropionitrile as solvent, in the presence of 0.1 to 5 mol.% of copper powder, copper bronze, copper(I) or copper(II) chloride or bromide or copper(I) iodide, or of a mixture of these substances, in the temperature range from 130° to 200°C, in a closed system, under a pressure corresponding to the respective reaction temperature.

17. A compound of the formula II

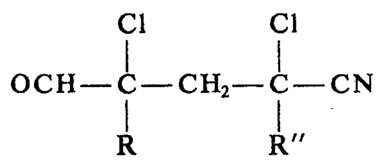

(II)

wherein either R is chlorine and R'' is methyl or trifluoromethyl, or R and R'' are methyl.

18. A process for the production of a compound of the formula II according to claim 17, which process comprises the addition of

a) trichloroacetaldehyde to methylacrylonitrile or $\alpha$-trifluoromethylacrylonitrile, or
b) 2,2-dichloropropionaldehyde to methacrylonitrile,

in the presence of a catalyst.

**Revendications**

1. Procédé de préparation des chloropyridines de formule (I):

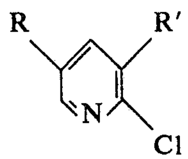

(I)

dans laquelle ou bien R représente le chlore et R' un groupe méthyle ou trifluorométhyle, ou bien R et R' représentent des groupes méthyle, caractérisé en ce que l'on fixe par addition, en présence d'un catalyseur

a) le trichloracétaldéhyde sur le méthacrylonitrile ou l'$\alpha$-trifluorométhylacrylonitrile ou bien
b) le 2,2-dichloropropionaldéhyde sur le méthacrylonitrile,

et on convertit le produit intermédiaire formé de formule (II):

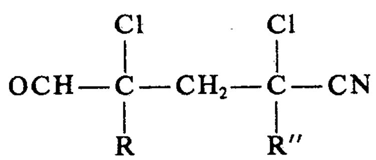

(II)

dans laquelle ou bien R représente le chlore et R'' un groupe méthyle ou trifluorométhyle; ou bien R et R'' représentent des groupes méthyle, par cyclisation en un composé de formule (I).

2. Procédé selon la revendication 1, caractérisé en ce que l'on effectue les réactions d'addition à une température de 70 à 160°C.

3. Procédé selon la revendication 1, caractérisé en ce que l'on effectue les réactions d'addition dans un système clos à une température de 70 à 160°C et une pression correspondant à la température de réaction observée.

4. Procédé selon la revendication 1, caractérisé en ce que l'on cyclise les composés de formule (II) à une température de 0 à 220°C.

5. Procédé selon la revendication 1, caractérisé en ce que l'on cyclise les composés de formule (II) à une température de 100 à 200°C dans un système ouvert en présence de chlorure d'hydrogène ou en présence de substances formant du chlorure d'hydrogène dans les conditions de la réaction.

6. Procédé selon la revendication 1, caractérisé en ce que l'on cyclise les composés de formule (II) en l'absence de solvant par chauffage en phase liquide ou gazeuse.

7. Procédé selon la revendication 1, caractérisé en ce que l'on isole d'abord les composés de formule (II) puis on les cyclise dans un deuxième stade opératoire.

8. Procédé selon la revendication 1, caractérisé en ce que l'on convertit directement en un composé de formule (I), en présence d'un catalyseur:

a) le trichloracétaldéhyde et le méthacrylonitrile ou l'$\alpha$-trifluorométhacrylonitrile ou
b) le 2,2-dichloropropionaldéhyde et le méthacrylonitrile,

sans isoler le composé de formule (II) formé en produit intermédiaire.

9. Procédé selon la revendication 8, caractérisé en ce que l'on effectue la réaction à une température de 70 à 220°C.

10. Procédé selon la revendication 8, caractérisé en ce que l'on effectue la réaction en système clos à une pression correspondant à la température de réaction.

11. Procédé selon la revendication 1 ou 8, caractérisé en ce que l'on utilise en tant que catalyseur de la poudre de cuivre, du bronze de cuivre, du chlorure ou bromure cuivreux ou cuivrique, de l'iodure cuivreux ou leurs mélanges.

12. Procédé selon la revendication 1 ou 8, caractérisé en ce que l'on utilise le catalyseur en quantité de 0,01 à 10 moles % par rapport à l'aldéhyde.

13. Procédé selon la revendication 1 ou 8, caractérisé en ce que l'on procède à la réaction d'addition ou à la conversion directe en composés de formule (I) en présence d'un solvant organique inerte.

14. Procédé selon la revendication 1 ou 8, caractérisé en ce que l'on procède aux réactions d'addition ou à la conversion directe en composés de formule (I) dans un nitrile d'acide alcanoïque en $C_2$—$C_5$, un 3-alcoxypropionitrile contenant 1 ou 2 atomes de carbone dans le groupe alcoxy ou en excès de méthacrylonitrile ou d'$\alpha$-trifluorométhacrylonitrile servant de solvant.

15. Procédé selon la revendication 1 ou 13, caractérisé en ce que l'on procède aux réactions d'addition à une température de 70 à 160°C dans l'acétonitrile, le butyronitrile ou le 3-méthoxypropionitrile servant de solvant en présence de 0,1 à 5 moles % de poudre de cuivre, de bronze de cuivre, de chlorure ou bromure cuivreux ou cuivrique ou d'iodure cuivreux ou en présence d'un mélange de ces substances dans un système clos et on cyclise en un composé de formule (I) le composé obtenu répondant à la formule (II) à une température de 100 à 200°C dans un système ouvert en présence de chlorure d'hydrogène ou d'une substance formant du chlorure d'hydrogène dans les conditions de la réaction.

16. Procédé selon la revendication 8 ou 13, caractérisé en ce que l'on effectue la réaction dans l'acétonitrile, le butyronitrile ou le 3-méthoxypropionitrile servant de solvant en présence de 0,1 à 5 moles % de poudre de cuivre, de bronze de cuivre, de chlorure ou bromure cuivreux ou cuivrique ou d'iodure cuivreux ou d'un mélange de ces substances à une température de 130 à 200°C en système clos à une pression correspondant à la température de réaction observée.

17. Composés de formule (II):

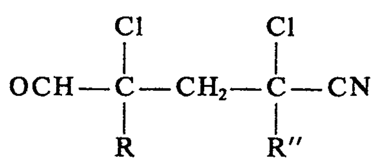

(II)

dans laquelle ou bien R représente le chlore et R'' un groupe méthyle ou trifluorométhyle, ou bien R et R'' représentent des groupes méthyle.

18. Procédé de préparation d'un composé de formule (II) selon la revendication 17, caractérisé en ce que l'on fixe par addition, en présence d'un catalyseur:

a) le trichloracétaldéhyde sur le méthacrylonitrile ou l'$\alpha$-trifluorométhylacrylonitrile, ou bien
b) le 2,2-dichloropropionaldéhyde sur le méthacrylonitrile.